# EUROPEAN PATENT APPLICATION

(11) **EP 2 377 933 A1**
(43) Date of publication of application: **19.10.2011**
(21) Application number: 09834071.4
(22) Date of filing: 10.12.2009
(51) Int. Cl.: C12N 15/11, C12Q 1/68

(54) **PROBES, LIQUID PHASE CHIPS AND METHODS FOR DETECTING PIK3CA GENE MUTATIONS**

(30) Priority: 23.12.2008 CN 200810220266
(71) Applicant: Guangzhou Surexam Bio-Tech Co., Ltd., Guangdong 510663 (CN)
(72) Inventor: XU, Jiasen, Guangzhou Guangdong 510663 (CN); GUO, Yuanjie, Guangzhou Guangdong 510663 (CN)
(74) Representative: Simons, Amanda Louise
(86) International application number: PCT/CN2009/075451
(87) International publication number: WO 2010/072118

(57) **Abstract**

Probes, liquid chips and methods for detecting PIK3CA gene mutations are provided, wherein the liquid chips for detecting PIK3CA gene mutations mainly comprise: microspheres coupled with probes; primers used for amplifying target sequences with exon 9 and/or exon 20. The liquid chips and methods for detecting PIK3CA gene mutations are useful for detecting the sites containing mutations of the PIK3CA gene with relatively high frequency simultaneously, and also are useful for detecting the exon 9 and exon 20 separately or simultaneously. The detection methods have identical reaction conditions of detection, good specificity of detection, high sensitivity, above 90% accuracy, and short time of detection.

## Description

### FIELD OF THE INVENTION

The present invention relates to the field of molecular biology, and in particular to probes, liquid chips and methods for detecting PIK3CA gene mutations.

### BACKGROUND OF THE INVENTION

The PIK3CA gene, coding for the catalytic subunit p110α of class IA phosphatidylinositol 3-kinases (PI3Ks), is the cellular homolog of the retroviral v-p3k oncogene. The class I PI3Ks are heterodimers, consisting of a p110 catalytic subunit and a p85 regulatory subunit, and can be activated by receptor tyrosine kinases such as insulin receptor and epidermal growth factor receptor (EGFR). Activated PI3Ks phosphorylate the phosphatidylinositol (4,5)-bisphosphate (PIP2) to generate the important intracellular second messenger phosphatidylinositol-3,4,5-trisphosphate (PIP3). PIP3 can activate the AKT pathway to induce a wide range of biological effects including regulation of cell proliferation, cell survival and cell cycle control, etc, relating to numerous targets molecules including mTOR (target of rapamycin), BAD, Caspase 9, Tuberin, GSK3β and a subset of fork head transcription factors, etc.

According to sequence analysis to the exon coding region of kinase domain of all the sixteen members of PI3K family, it is found that PIK3CA gene is the only oncogene caused by somatic mutation. Further study shows that PIK3CA mutations occur mainly in the helical domain and the catalytic domain, called as hot spot mutations of the PIK3CA gene. Recent studies show that mutations in the PIK3CA gene have been identified in several types of cancer including breast cancer, oophoroma, colon canre, rectal cancer, lung cancer, liver cancer, gastric cancer, etc. Currently, the PI3K signaling pathway is the main target in developing anticancer small molecule inhibitors in many pharmaceuticals companys, and the PIK3CA gene is also proved to be the main target site in cancer diagnosis and treatment.. At present, several drugs such as Quinolone, Pyridopyrimidine and Imidazopyridine are developed according to the PIK3CA target site and the corresponding PI3K signaling pathway. However, due to the high frequency of mutations of the PIK3CA gene in human cancers, there are individual differences in the therapeutic effects of drugs targeting at EGFR gene and PI3K pathway. Research from Maruyama et al finds that the prognosis is more positive in patients with PIK3CA gene mutation. Some researchers pointed out that the prognosis for breast cancer patients with different mutation sites in the PIK3CA gene is different. The prognosis for patients with exon 9 mutations (helical domain) is poorer than that for wild type individuals, while exon 20 mutations (catalytic domain) has a more positive prognosis than wild type individuals. Although there is no conclusion on the prognosis for cancer patient by the PIK3CA somatic mutation, all these researches indicate that the PIK3CA gene mutation is an important factor affecting the prognosis. Therefore, for cancer patients especially breast cancer patients, it is necessary to detect the PIK3CA somatic mutation so as to achieve the personalized therapy of cancer.

At present, the main method for detection of PIK3CA somatic mutation is direct sequencing on tissue DNA samples. The direct sequencing method has advantages such as directness and direct viewing. With this method, the mutation sites and mutant-types of the PIK3CA gene can be exactly known. However, the direct sequencing method is poor in sensitivity and low in efficiency, which restricts its clinical application greatly. For the tumor tissue has heterogeneity, a large number of wild type genes are also contained in patients with PIK3CA somatic mutations, which interferes with the effective detection of PIK3CA somatic mutations seriously. Currently, the detection sensitivity of the direct sequencing method can reach about 15% to 25%. Meanwhile, it is difficult for the direct sequencing method to achieve high-throughput detection, and its chronergy restricts its clinical application greatly. The site-specific Real-time PCR method is the most advanced technique for detection of PIK3CA gene mutations in the world. This method improves the sensitivity and chronergy of detection. However, the sit-specific Real-time PCR method has high false positive rate, i.e. poor specificity, and this has attracted many critics in clinical diagnosis.

Accordingly, if the detection of PIK3CA gene mutation will become a normal clinical detection technique, a new detection technique with high sensitivity and good specificity and easy in operation and can achieve high-throughput detection should be established, whereby hospitals at all levels can carry out this gene diagnosis project.

### SUMMARY OF THE INVENTION

It is an object of the present invention to provide probes for detecting PIK3CA gene mutations, and a liquid chip for detecting PIK3CA gene mutations prepared by using these probes. The liquid chip can be used to detect the somatic mutant of PIK3CA exon 9 and/or exon 20, and provide a suggestion for clinical treatment.

The above object is achieved by the following technical solutions:

Probes for detecting PIK3CAgene mutations, comprise:
a wild-type probe of SEQ ID NO.1 specific for E542, and a probe of SEQ ID NO.2 specific for E542K point mutation; and/ or a wild-type probe of SEQ ID NO.3 specific for E545, a probe of SEQ ID NO.4 specific for E545K point mutation, and a probe of SEQ ID NO.5 specific for E545D point mutation; and/ or a wild-type probe of SEQ ID NO.6 specific for H1047, and a probe of SEQ ID NO.7 specific for H1047R point mutation.

A liquid chip for detecting PIK3CA gene mutations, comprises:
microspheres coupled with amino-substituted wild-type probes of SEQ ID NO.1 specific for E542, and microspheres coupled with amino-substituted probes of SEQ ID NO.2 specific for E542K point mutation; and/ or microspheres coupled with amino-substituted wild-type probes of SEQ ID NO.3 specific for E545, microspheres coupled with amino-substituted probes of SEQ ID NO.4 specific E545K point mutation, and microspheres coupled with amino-substituted probes of SEQ ID NO.5 specific E545D point mutation; and/ or microspheres coupled with amino-substituted wild-type probes of SEQ ID NO.6 specific for H1047, microspheres coupled with amino-substituted probes of SEQ ID NO.7 specific for H1047R point mutation;
wherein a spacer is connected between the nucleotide sequence of each kind of the above-mentioned probes and the amino group, and microspheres coupled with different probes have different color codes; and
primers for amplifying target sequences enriched for mutant alleles of PIK3CA exons 9 and/or 20, the target sequence being biotin-labeled at the terminal.

Preferably, the primers for amplifying target sequences enriched for mutant alleles of PIK3CA exon 9 comprises SEQ ID NO.8-SEQ ID NO.10, and at least one of the primers is biotin-labeled at the terminal; and/ or the primers for amplifying target sequences enriched for mutant alleles of PIK3CA exon 20 comprises SEQ ID NO.11-SEQ ID NO.13, and at lease one of the primers is biotin-labeled at the terminal.

It is a further object of the present invention to provide a method for detecting PIK3CA gene mutations, which is rapid and accurate in detection, easy in operation, and can detect multiple mutation alleles simultaneously.

A method for detecting PIK3CA gene mutations, using the above-mentioned liquid chip, comprises the following steps:
1) extracting the DNA from the samples, and performing a first PCR amplification for DNA samples by using the primers for amplifying target sequences enriched for mutant alleles of PIK3CA exons 9 and/or 20;
2) performing restriction digestion to products obtained from the first PCR amplification;
3) performing a second PCR amplification by using products obtained after the restriction digestion as template;
4) hybridizing products obtained from the second PCR amplification to the corresponding probes coupled to above-mentioned microspheres;
5) performing reaction by adding streptavidin-phycoerythrin after the hybridization reaction of the step (4), and then performing signal detection using the Luminex instrument.

Preferably, the primers used in the first PCR amplification in step 1) are: SEQ ID NO.8 and SEQ ID NO.9; and/ or SEQ ID NO.11 and SEQ ID NO.12; the primers used in the second PCR amplification in step 3) are: SEQ ID NO.9 and SEQ ID NO.10; and/ or SEQ ID NO.12 and SEQ ID NO. 13.

Preferably, the hybridization temperature is 55-60° C.

Preferably, each kind of the microspheres coupled with the probes is prepared through a method comprising the following steps:
(1) suspending a stock uncoupled microspheres;
(2) transferring 8ul of the stock microspheres, which contains a total of 0.8×10⁵ to 1.2×10*⁵* microspheres, to a 0.5ml microcentrifuge tube;
(3) pelleting the microspheres by microcentrifugation at ≥10,000 rpm for 2 to 5 min, and remove the supernatant;
(4) adding 10ul of coupling solution, and mixing evenly by vortex;
(5) adding 2ul of 2pmol/ul probe working solution;
(6) adding 2.5ul of 5mg/ml EDC working solution, and incubating at 25 °C for 30min; and repeating this step once;
(7) adding 0.2ml of washing buffer, mixing evenly by vortex and then pelleting by microcentrifugation at ≥10,000rpm for 2 to 5min, and removing the supernatant; repeating this step once;
(8) adding 500µl of TE buffer, and mixing evenly by vortex;
(9) pelleting by microcentrifugation at ≥10,000 rpm for 2 to 5 min, and removing the supernatant;
(10) adding 17µl of TE buffer, and mixing evenly by vortex to obtain a coupled microspheres with concentration of approximately 5×10³ microspheres per µl;
(11) counting the coupled micorspheres by transferring 2µl microspheres diluting 50 times in dH₂O, storing the coupled microspheres at 2-8 °C.

According to the current studies, PIK3CA mutations occur mainly in the exon 9 (helical domain) and the exon 20 (catalytic domain), in particular, amino acid sites E542, E545 and H1047 are called as mutation hot spots in PIK3CA gene, and this occupies about 80% of the PIK3CA point mutations. Therefore, E542, E545 and H1047 which are highest in mutation rate are selected for mutation detection in the present invention.

The present invention has the following advantages:
(1) By using the liquid chip for detecting PIK3CA gene mutations, the alleles with a relatively high mutation frequency in PIK3CA gene can be simultaneously or respectively detected, the reaction condition is uniform during detection, the steps are easy in operation, and the detection chronergy is far more better than the direct sequencing technique, meanwhile the parallel detection of multiple mutation sites can achieve the high-throughput detection.
(2) The present invention uses the method of introducing restriction site and then enriching by restriction digestion so as to amplify the target sequence and then to be used in detection, which prevents the large amount of wild-type sequences in the products from disturbing the detection results. This improves the sensitivity of detection greatly, and the detection techniques in prior art are unmatchable.
(3) The method of introducing restriction site and the liquid chip are effectively combined in the present invention, which avoids high false positive rate and improves the specificity and accuracy.
(4) The probes of the present invention have the uniform reaction condition in the hybridization reaction, and there is almost no non-specific combination among the probes. The probes have good specificity and high signal to noise ratio in the detection. Meanwhile, a nice system with good detection results is formed by using the liquid chip and detection method.

### DETAILED DESCRIPTION OF THE PREFERRED EMBODIMENT

The spacer is the sequence used for separating the specific probe from the microsphere surface or placing the specific probe into the hydrophilic environment. By providing an appropriate length of spacer sequence between the probe sequence and the amino group, the steric hindrance is reduced, and the efficiency of hybridization and the specificity of hybridization are improved. The familiar spacer sequences include poly (dT), oligomer polyethylene glycol and (CH2)n spacer (n≥3), such as (CH2) 12, (CH2) 18, etc. In addition, if the disturbing of poly (dA) exists, it can also use the poly (TTG) to be the spacer. Preferably, the spacer is an oligonucleotide consisting of 5 to 30 deoxythymidylates, and more preferably 10 deoxythymidylates. In China, the synthetic technique for deoxythymidylates is well developed, and the cost is relatively low.

### Solution Formulation:

Coupling buffer (pH4.5): 0.1mol/LMES (Sigma M-2933)
Washing buffer: 0.2ml/L Tween-20 (Sigma P-9416), 1g/LSDS (Sigma L-4390)
TE buffer (pH8.0) (Storage solution): 10mmol/L Tris (Sigma 337501), 1mmol/L EDTA (Sigma E-5134)
2×Tm hybridization buffer

| Reagent | Source | Final concentration | Serving size per 250ml |
|---|---|---|---|
| 1MTris-HCl, pH8.0 | SigmaT3038 | 0.2M | 50ml |
| 5M NaCl | Sigma S5150 | 0.4M | 20ml |
| Triton X-100 | Sigma T8787 | 0.16% | 0.4ml |

Filter and stored at 4 °C.
1 × Tm hybridization buffer

| Reagent | Source | Final concentration | Serving size per 250ml |
|---|---|---|---|
| 1MTris-HCl, pH8.0 | SigmaT3038 | 0.1M | 25ml |
| 5M NaCl | Sigma S5150 | 0.2M | 10ml |
| Triton X-100 | Sigma T8787 | 0.08% | 0.2ml |

Filter and stored at 4 °C.

### EMBODIMENT 1

### Preparation of liquid chip for detecting PIK3CA gene mutations

### 1. The probes and microsphere coupling

Specific oligonucleotide probes were designed for detection of the wild-type and mutant-type of PIK3CA exons 9 and/or 20. The 5' terminal of the probe is connected with an amino group through a spacer with 10 deoxythymidylates. The probes are synthesized by Sangon Biotech (Shanghai) Co., Ltd. The probes are respectively coupled with microspheres (purchased from Luminex Corporation) of different color codes by covalent coupling.

The coupling of each kind of the microspheres comprises the following steps:
(1) resuspend the stock uncoupled microspheres (purchased from Luminex Corporation) by vortex;
(2) transfer 8µl of the stock microspheres, which contains a total of 0.8×10⁵ to1.2×10⁵ microspheres, to a 0.5ml microcentrifuge tube;
(3) pellet the microspheres by microcentrifugation at 10,000 rpm for 2 min, and remove the supernatant;
(4) add 10µl of coupling solution (pH 4.5), and mix evenly by vortex;
(5) add 2µl of 2pmol/ul probe working solution;
(6) add 2.5µl of 5mg/ml EDC (1-(3-Dimethylaminopropyl)-3-ethylcarbodiimide hydrochloride) working solution, and incubate at 25 °C for 30min; and repeat this step once;
(7) add 0.2ml of washing buffer, mix evenly by vortex and then pellet by microcentrifugation at 10,000rpm for 2 min, and remove the supernatant; repeat this step once;
(8) add 500µl of TE buffer, and mix evenly by vortex;
(9) pellet by microcentrifugation at 10,000 rpm for 2 min, and remove the supernatant;
(10) add 20µl of TE buffer, and stored at 2-8 °C.

The nucleotide sequence for each probe is as follows:

| SEQID.NO | Name | Nucleotide Sequences <NH₂+poly (dT) +SEQ) |
|---|---|---|
| 1 | E542-P | 5'NH2-tttttttttt    TACACGAGATCCTCTCTCTGAA 3' |
| 2 | E542K-P | 5'NH2-tttttttttt TACACGAGATCCTCTCTCTAAA 3' |
| 3 | E545-P | 5'NH₂-tttttttttt TCACTGAGCAGGAGAAAGATT3' |
| 4 | E545K-P | 5'NH2-tttttttttt TCACTAAGCAGGAGAAAGATT 3' |
| 5 | E545D-P | 5'NH₂-tttttttttt TCACTGATCAGGAGAAAGATT 3' |
| 6 | H1047-P | 5'NH₂-tttttttttt GATGCACATCATGGTGGCTG |
| 7 | H1047R-P | 5'NH₂-tttttttttt GATGCACGTCATGGTGGCTG |
| 14 | N-P | 5'NH₂-tttttttttt CGTTAGGTCGGAGCTTGATC |

### Primer design and labeling

For the nucleotide sequence of PIK3CA gene exons 9 and 20, the primers are designed as follows:

| Primers name | SEQ ID.NO | Nucleotide sequences (5'→3', bp) | Size (bp) |
|---|---|---|---|
| PE9-AS1 | 8 | GGAACTTTACCACACTGCTGAACC | 221 |
| PE9-S1 | 9 | GCAGGAGAAAGATTTTCTATGG | |
| PE9-AS2 | 10 | CTTCTCGGGATACAGACCAAT | 167 |
| PE9-S1 | 9 | GCAGGAGAAAGATTTTCTATGG | |
| PE20-AS1 | 11 | CCTTGTAACACATCTCCTG | 252 |
| PE20-S1 | 12 | GGATCTTCCACACAATTAAACAG | |
| PE20-AS2 | 13 | ATTGTAGTTCTGGCATTCC | 168 |
| PE20-S1 | 12 | GGATCTTCCACACAATTAAACAG | |

The primers are synthesized by Sangon Biotech (Shanghai) Co., Ltd. The 5' terminals of PE9-AS2 and PE20-AS2 are respectively biotin-labeled. PE9-S1 and PE9-AS1 are used in the first PCR amplification of exon 9, PE9-S1 and the biotin-labeled PE9-AS2 are used in the second PCR amplification of exon 20; PE20-S1 and PE20-AS1 are used in the first PCR amplification of exon 20, PE20-S1 and the biotin-labeled PE20-AS2 are used in the second PCR amplification of exon 20.

The obtained liquid chip for detecting PIK3CAgene mutations, comprises:
microspheres coupled with amino-substituted wild-type probes of SEQ ID NO.1 specific for E542, and microspheres coupled with amino-substituted probes of SEQ ID NO.2 specific for E542K point mutation; microspheres coupled with amino-substituted wild-type probes of SEQ ID NO.3 specific for E545, microspheres coupled with amino-substituted probes of SEQ ID NO.4 specific for E545K point mutation, and microspheres coupled with amino-substituted probes of SEQ ID NO.5 specific for E545D; and
microspheres coupled with amino-substituted wild-type probes of SEQ ID NO.6 specific for H1047, microspheres coupled with amino-substituted probes of SEQ ID NO.7 specific for H1047R point mutation;
wherein a spacer is connected between the nucleotide sequence of each kind of the above-mentioned probes and the amino group, and microspheres coupled with different probes have different color codes; and
the primers comprise SEQ ID NO.8-SEQ ID NO.10 and SEQ ID NO.11-SEQ ID NO.13, and the primers SEQ ID NO.10 and SEQ ID NO.13 are biotin-labeled at the 5' terminal.

### Embodiment2

Breast cancer tissue samples are detected by using the liquid chip for detecting PIK3CA gene mutations of the Embodiment 1.

### 1. Preparation of the samples to be detected

Extraction of DNA from the breast cancer tissue samples: 5-50 mg of breast cancer issue samples are ground and then washed twice by using PBS buffer; the washed tissue samples are resuspended in 1ml of the digestion buffer (50mmol/L Tris, 1mmo/L Na₂EDTA, 0.5 % Tween-20, 200ug/ml of Proteinase K 200, pH 8.5), and placed in 55°C water bath to digest for 1 hour, and then placed in 99°C water bath to inactivate the proteinase K; centrifuge the tube at 12000 rpm for 10 minutes; remove the supernatant, extracted with phenol-chloroform-isopentanol, and obtain the DNA samples for PCR reaction through ethanol precipitation method. The DNA may also be extracted by microspincolumn method.

### 2. Enrichment by restriction digestion and PCR amplification of the samples to be detected

### (1) Enrichment by restriction digestion and PCR amplification of the DNA samples

The enrichment for mutant-type of E545 and E542 on exon 9 by restriction digestion and PCR amplification is as follows:

### 1). The first PCR amplification

### PCR reaction system

| | |
|---|---|
| 10×TureStart DNA polymerase Buffer | 2.5µl |
| 2.5mM dNTP Mixture | 2µl |
| PE9-AS1 | 0.5µl (20µmol/L) |
| PE9-S1 | 0.5µl (20µmol/L) |
| TureStart Taq DNA polymerase | 0.5µl (2U/µl) |
| DNA Template | 1µl |

Add double-distilled (dd) water up to 25µl

### PCR Reaction Condition:

| Steps | Temperature | Time | Cycle number |
|---|---|---|---|
| Initial denaturation | 95 °C | 5 min | 1 |
| Denaturation | 95 °C | 30 sec | |
| Anneal | 58°C | 30 sec | 20 |
| Extension | 72 °C | 45 sec | |
| Final Extension | 72 °C | 10 min | 1 |

### 2). Restriction digestion of PCR products:

Reaction system of E542 Restriction Digestion:

| | |
|---|---|
| 10×NEB Buffer 4 | 1µl |
| PCR Products | 3µl |
| Hpy188 I | 0.5µl (10U/µl) |
| Add double-distilled (dd) water up to | 10µl |

Incubate at 37°C for 2 hours, and inactivate enzyme at 65°C for 20 minutes.

### Reaction system of E545 Restriction Digestion:

| | |
|---|---|
| 10×NEB Buffer 4 | 1µl |
| PCR Products | 3µl |
| TspR I | 0.5µl (10U/µl) |
| 100×BSA | 0.1ul |
| Add double-distilled (dd) water up to | 10µl |

Incubate at 65°C for 2 hours.

### 3). The second PCR amplification

### PCR reaction system:

| | |
|---|---|
| 10×TureStart DNApolymerase Buffer | 2.5µl |
| 2.5mMdNTP Mixture | 2µl |
| PE9-S1 | 0.5µl (20µmol/L) |
| Biotin-PE9 -As2 | 0.5µl (20µmol/L) |
| TureStart Taq DNA polymerase | 0.5µl (2U/µl) |
| Restriction Digestion Products | 1µl |
| Add double-distilled (dd) water up to | 25µl |

### PCR reaction condition:

| Steps | Temperature | Time | Cycle Number |
|---|---|---|---|
| Initial denaturation | 95 °C | 5 min | 1 |
| Denaturation | 95 °C | 30 sec | |
| Anneal | 59 °C | 30 sec | 30 |
| Extension | 72 °C | 45sec | |
| Final Extension | 72 °C | 10 min | 1 |

The enrichment for mutant-type of H1047 on exon 20 by restriction digestion and PCR amplification is as follows:

### 1). The first PCR amplification

### PCR reaction system

| | |
|---|---|
| 10×TureStart DNApolymerase Buffer | 2.5µl |
| 2.5mMdNTP Mixture | 2µl |
| PE20-S1 | 0.25µl (20µmol/L) |
| PE20-As1 | 0.25µl (20µmol/L) |
| TureStart Taq DNA polymerase | 0.5µl (2U/µl) |
| DNA Template | 1µl |
| Add double-distilled (dd) water up to | 25µl |

### PCR Reaction Condition:

| Steps | Temperature | Time | Cycle Number |
|---|---|---|---|
| Initial denaturation | 95 °C | 5 min | 1 |
| Denaturation | 95 °C | 30 sec | |
| Anneal | 53 °C | 30 sec | 20 |
| Extension | 72 °C | 45 sec | |
| Final Extension | 72 °C | 10 min | 1 |

### 2). BsaBI restriction digestion of PCR products:

Reaction system is as follows:

| | |
|---|---|
| 10× NEB Buffer 2 | 1µl |
| PCR Products | 3µl |
| BsaBI | 0.5µl (10U/µl) |
| Add Nuclease-Free Water up to | 10µl |

Incubate at 60°C for 2 hours, and inactivate enzyme at 80°C for 20 minutes.

### 3). The second PCR amplification

### PCR reaction system:

| | |
|---|---|
| 10×TureStart DNApolymerase Buffer | 2.5µl |
| 2.5mM dNTP Mixture | 1µl |
| Biotin-PE20-As2 | 0.5µl (20µmol/L) |
| PE20-S1 | 0.5µl (20µmol/L) |
| TureStart Taq DNA polymerase | 0.5µl (2U/µl) |
| Restriction Digestion Products | 1µl |
| Add double-distilled (dd) water up to | 25µl |

### PCR Reaction Condition:

| Steps | Temperature | Time | Cycle Number |
|---|---|---|---|
| Initial denaturation | 95 °C | 5 min | 1 |
| Denaturation | 95 °C | 30 sec | |
| Anneal | 53 °C | 30 sec | 30 |
| Extension | 72 °C | 45 sec | |
| Final Extension | 72 °C | 10 min | 1 |

### 3. Hybridization and detection

(1) Microspheres coupled with E542P, E542K-P, E545-P, E545K-P, E545D-P, H1047-P, H1047R-P, and negative control probes are respectively mixed by vortex;
(2) 1.5×Tm hybridization buffer is used to prepare the working solution of microspheres mixture coupled with probes such that each kind of microspheres in the working solution has a concentration of 75 microspheres per µl;
(3) The working solution of microspheres mixture is mixed evenly by vortex;
(4) 33 µl of the working solution of microspheres mixture is added into each well to allow the reaction system to contain about 2000 to 2500 microspheres of each kind;
(5) 17 µl TE buffer (pH8.0) is added into the background well; each of the other wells is added into 2-17 µl of the second PCR amplification products of each sample, TE buffer is further added up to 17 µl; mix gently; cover the reaction plate (tube cover) to prevent evaporation;
(6) Set the PCR programe to: 95°C, 5min; hybridisation incubation at 60 °C for 15min;
(7) 1 × Tm hybridization buffer is used to prepare SA-PE up to 10ug/ml (25ul/well);
(8) Add 25ul of SA-PE (streptavidin phycoerythrin) working solution to each well;
(9) Place at hybridization temperature of 60 °C immediately and incubate for 5min;
(10) Set the Luminex instrument to the hybridization temperature, and read the data.

### 4. Detection results and data analysis

The reaction products are detected by using the Luminex analyzer. The detection results are shown in Table 1. Before this, the wild-type DNA samples (non-specific) are detected, the fluorescent value of each detection is recorded, and the mean value of the fluorescent values of the microspheres with mutant-type probes is added with 5 times standard deviation (Mean+5*SD) to be used as the cut-off value of each mutation detection. The cut-off values of E542K-P, E545K-P, E545D-P and H1047R-PA obtained according to this method are all close to 100. Therefore, the cut-off values of the probes of this liquid chip for detecting PIK3 CA gene mutations are determined as 100.

According to this determination, in 10 samples detected in this embodiment, 5 samples has point mutation, wherein the mutant-type of Sample No. 2 is E542K, the mutant-type of Sample No. 3 is E545D, the mutant-type of Samples No. 5 and 8 is H1047R, and the mutant-type of Sample No. 9 is E542K.

Meanwhile, the samples are detected according to sequencing detection (see Table 2), wherein it is difficult to determine whether the Sample No. 2 has mutations by using sequencing detection (undesired peaks appear in the sequencing, it can be seen from the detection results of the liquid chip, the probe E545-P also has response, the MFI value is 787, while the detection value of the probe E545K-P is 209, and this indicates that there is heterogeneity in this sample and it contains relatively low proportion of the mutant-type DNA). The sequencing results of the other 9 samples are identical to the result as determined above in this embodiment, and thus the coincidence rate reaches 100%. Even taking account of the sample that can not be determined, the coincidence rate also reaches 90%. This assay also indicates that the liquid chip for detecting PIK3CA gene mutations and the detection method can detect the mutant-types and also have high accuracy and specificity in detection, and the detection sensitivity is obviously better than the sequencing detection (the Sample No. 2 can not be determined by the sequencing method).

**Table 1: Detection results of the sample**

| Sample NO. | Negative Control (MFI) | E542-P (MFI) | E542K-P (MFI) | E545-P (MFI) | E545K-P (MFI) | E545D-P (MFI) | H1047-P (MFI) | H1047R-P (MFI) |
|---|---|---|---|---|---|---|---|---|
| 1 | 27 | 1223 | 28 | 1434 | 15 | 41 | 1275 | 69 |
| 2 | 13 | 1304 | 35 | 787 | 209 | 22 | 1453 | 27 |
| 3 | 18 | 1442 | 28 | 1254 | 63 | 951 | 1132 | 41 |
| 4 | 7.5 | 1251 | 26 | 1534 | 15 | 33 | 1417.5 | 43 |
| 5 | 23 | 1534 | 35.5 | 1221 | 45 | 18.5 | 428.5 | 754 |
| 6 | 14 | 1147.5 | 41 | 1332 | 37 | 25 | 1041 | 24 |
| 7 | 19.5 | 1531 | 26 | 1121.2 | 36 | 12 | 1245 | 27 |
| 8 | 22 | 1436 | 51 | 1426 | 42.5 | 15 | 117 | 581 |
| 9 | 8.5 | 520.5 | 1171 | 1234 | 21 | 43.5 | 1316 | 18.7 |
| 10 | 13.5 | 14 | 27 | 1423 | 28.5 | 14.5 | 1136 | 41 |

**Table 2: Comparative analysis of the detection results of samples**

| Sample No. | Detection results of the liquid chip | | | Sequencing results | | |
|---|---|---|---|---|---|---|
| | 1624 | 1633 | 3140 | 1624 | 1633 | 3140 |
| 1 | Wild-type | Wild-type | Wild-type | Wild-type | Wild-type | Wild-type |
| 2 | Wild-type | Mutant-type (G1633A) | Wild-type | Wild-type | Can not determine from peakvalue diagram | Wild-type |
| 3 | Wild-type | Mutant-type (G1633T) | Wild-type | Wild-type | Mutant-type (G1633T) | Wild-type |
| 4 | Wild-type | Wild-type | Wild-type | Wild-type | Wild-type | Wild-type |
| 5 | Wild-type | Wild-type | Mutant-type (A3140G) | Wild-type | Wild-type | Mutant-type (A3140G) |
| 6 | Wild-type | Wild-type | Wild-type | Wild-type | Wild-type | Wild-type |
| 7 | Wild-type | Wild-type | Wild-type | Wild-type | Wild-type | Wild-type |
| 8 | Wild-type | Wild-type | Mutant-type (A3140G) | Wild-type | Wild-type | Mutant-type (A3140G) |
| 9 | Mutant-type (G1624A) | Wild-type | Wild-type | Mutant-type (G1624A) | Wild-type | Wild-type |
| 10 | Wild-type | Wild-type | Wild-type | Wild-type | Wild-type | Wild-type |

### Embodiment 3, Detection sensitivity test for the liquid chip for detecting PIK3CA gene mutations

In order to test the detection sensitivity of the liquid chip, it is designed to determine the capability to detect the PIK3CA wild-type DNA from the mutant-type DNA, that is, to determine the minimum ratio of the mutant-type DNA that the liquid chip can detect. In this test, the copy number of the wild-type DNA, the mutant-type DNA in each group is shown in Table 3. The test of each group is repeated several times. In this embodiment, the first PCR amplification, the restriction digestion, the second PCR amplification and the Luminex detection are identical to that of the Embodiment 2.

**Table 3, Test results of the sensitivity of the liquid chip for detecting PIK3 CA gene mutations**

| Wild-type Copy Number | Mutant-type DNA Copy Number | Percentage of Mutant-type DNA | E542K-P (MFI) | E545K-P (MFI) | E545D-P (MFI) | H1047R-P (MFI) |
|---|---|---|---|---|---|---|
| 500 | 5 | 1% | 778 | 695 | 621 | 867 |
| 1000 | 5 | 0.5% | 389 | 414 | 341 | 298 |
| 5000 | 5 | 0.1% | 204 | 153 | 178 | 193 |

In the table 3, the fluorescent intensity (MFI) values higher than 100 are determined as positive, which belongs to effective detection.

The test results show that: by using the liquid chip for detecting the PIK3CA gene mutations, the technique of enrichment by restriction digestion and the liquid chip technique are effectively combined, 5 copies of mutant-type DNA can be detected among 5000 copies of wild-type DNA, and the detection sensitivity is at least 0.1 %. Meanwhile, by using the detection method of this invention, at least 5 copies of mutant-type DNA can be detected.

## Claims

1. Probes for detecting PIK3CA gene mutations, comprising:
a wild-type probe of SEQ ID NO.1 specific for E542, and a probe of SEQ ID NO.2 specific for E542K point mutation; and/ or a wild-type probe of SEQ ID NO.3 specific for E545, a probe of SEQ ID NO.4 specific for E545K point mutation, and a probe of SEQ ID NO.5 specific for E545D point mutation; and/ or a wild-type probe of SEQ ID NO.6 specific for H1047, and a probe of SEQ ID NO.7 specific for H1047R point mutation.

2. A liquid chip for detecting PIK3CA gene mutations, comprising:
microspheres coupled with amino-substituted wild-type probes of SEQ ID NO.1 specific for E542, and microspheres coupled with amino-substituted probes of SEQ ID NO.2 specific for E542K point mutation; and/or microspheres coupled with amino-substituted wild-type probes of SEQ ID NO.3 specific for E545, microspheres coupled with amino-substituted probes of SEQ ID NO.4 specific for E545K point mutation, and microspheres coupled with amino-substituted probes of SEQ ID NO.5 specific for E545D point mutation; and/ or
microspheres coupled with amino-substituted wild-type probes of SEQ ID NO.6 specific for H1047, and microspheres coupled with amino-substituted probes of SEQ ID NO.7 specific for H1047R point mutation;
wherein a spacer is connected between the nucleotide sequence of each kind of the above-mentioned probes and the amino group, and microspheres coupled with different probes have different color codes; and
primers for amplifying target sequences enriched for mutant alleles of PIK3CA exons 9 and/or 20, and the target sequence being biotin-labeled at the terminal.

3. The liquid chip for detecting PIK3CA gene mutations of claim 2, wherein the spacer is an oligonucleotide consisting of 5 to 30 deoxythymidylates.

4. The liquid chip for detecting PIK3CA gene mutations of claim 2, wherein the primers for amplifying target sequences enriched for mutant alleles of exon 9 comprises SEQ ID NO.8-SEQ ID NO.10, and at least one of the primers is biotin-labeled at the terminal; and/ or the primers for amplifying target sequences enriched for mutant alleles of exon 20 comprises SEQ ID NO.11-SEQ ID NO.13, and at lease one of the primers is biotin-labeled at the terminal.

5. A method for detecting PIK3CA gene mutations, using the liquid chip for detecting PIK3CA gene mutations according to claim 2, comprising the following steps:
1) extracting the DNA from the samples, performing a first PCR amplification for DNA samples by using the primers for amplifying target sequences enriched for mutant alleles of PIK3CA exons 9 and/or 20;
2) performing restriction digestion to products obtained from the first PCR amplification;
3) performing a second PCR amplification by using products obtained after the restriction digestion as template;
4) hybridizing products obtained from the second PCR amplification to the corresponding probes coupled to microspheres of claim 2;
5) performing reaction by adding streptavidin-phycoerythrin after the hybridization reaction of the step (4), and then performing signal detection.

6. The method for detecting PIK3CAgene mutations of claim 5, wherein the primers used in the first PCR amplification in step 1) are: SEQ ID NO.8 and SEQ ID NO.9; and/ or SEQ ID NO.11 and SEQ ID NO.12; the primers used in the second PCR amplification in step 3) are: SEQ ID NO.9 and SEQ ID NO. 10; and/ or SEQ ID NO.12 and SEQ ID NO.13.

7. The method for detecting PIK3CA gene mutations of claim 5, wherein the hybridization temperature in the step 4) is 55-60° C.

8. The method for detecting PIK3CA gene mutations of claim 5, wherein each kind of the microspheres coupled with the probes is prepared through a method comprising the following steps:
(1) suspending the stock uncoupled microspheres;
(2) transferring 8µl of the stock microspheres, which contains a total of 0.8×10⁵ to 1.2×10⁵ microspheres, to a 0.5ml microcentrifuge tube;
(3) pelleting the stock microspheres by microcentrifugation at ≥10,000 rpm for 2 to 5 min, and remove the supernatant;
(4) adding 10ul of coupling solution, and mixing evenly by vortex;
(5) adding 2µl of 2pmol/ul probe working solution;
(6) adding 2.5µl of 5mg/ml EDC working solution, and incubating at 25 °C for 30min; and repeating this step once;
(7) adding 0.2ml of washing buffer, mixing evenly by vortex and then pelleting by microcentrifugation at ≥10,000rpm for 2 to 5min, and removing the supernatant; repeating this step once;
(8) adding 500µl of TE buffer, and mixing evenly by vortex;
(9) pelleting by microcentrifugation at ≥10,000 rpm for 2 to 5 min, and removing the supernatant;
(10) adding 17µl of TE buffer, and mixing evenly by vortex to obtain a coupled microspheres with a concentration of approximately 5×10³ microspheres per µl;
(11) counting the coupled micorspheres by transferring 2µl microspheres diluting 50 times in dH₂O, storing the coupled microspheres at 2-8 °C.


SEQUENCE LISTING

<110>  GUANGZHOU SUREXAM BIO TECH CO., LTD.

<120>  Probes, liquid phase chips and methods for detecting PIK3CA gene mutations

<130> N114480

<140> EP09834071.4
<141> 2009-12-10

<150> CN20081220266
<151> 2008-12-23

<160> 14

<170> PatentIn version 3.1

<210> 1
<211> 22
<212> DNA
<213> artificial sequence

<220>
<223> probe E542-P

<400> 1

tacacgagat cctctctctg aa                    22

<210> 2
<211> 22
<212> DNA
<213> artificial sequence

<220>
<223> probe E542K-P

<400> 2

tacacgagat cctctctcta aa                    22

<210> 3
<211> 21
<212> DNA
<213> artificial sequence

<220>
<223> probe E545-P

<400> 3

tcactgagca ggagaaagat t                     21

<210> 4
<211> 21
<212> DNA
<213> artificial sequence

<220>
<223> probe E545K-P

<400> 4

tcactaagca ggagaaagat t                     21

<210> 5
<211> 21
<212> DNA
<213> artificial sequence

<220>
<223> probe E545D-P

<400> 5

tcactgatca ggagaaagat t                                21

<210> 6
<211> 20
<212> DNA
<213>artificial sequence

<220>
<223> probe H1047-P

<400> 6

gatgcacatc atggtggctg                                  20

<210> 7
<211> 20
<212> DNA
<213> artificial sequence

<220>
<223> probe H1047R-P

<400> 7

gatgcacgtc atggtggctg                                  20

<210> 8
<211> 24
<212> DNA
<213> artificial sequence

<220>
<223> primer PE9-AS1

<400> 8
ggaactttac cacactgctg aacc                             24

<210> 9
<211> 22
<212> DNA
<213> artificial sequence

<220>
<223> primer PE9-S1

<400> 9

gcaggagaaa gattttctat gg                               22

<210> 10
<211> 21
<212> DNA
<213> artificial sequence

<220>
<223> primer PE9-AS2

<400> 10

cttctcggga tacagaccaa t                                21

<210> 11
<211> 19
<212> DNA
<213> artificial sequence

<220>
<223> primer PE20-AS1

<400> 11

ccttgtaaca catctcctg                                   19

<210> 12
<211> 23
<212> DNA
<213> artificial sequence

<220>
<223> primer PE20-S1

<400> 12

ggatcttcca cacaattaaa cag                              23

<210> 13
<211> 19
<212> DNA
<213> artificial sequence

<220>
<223> primer PE20-AS2

<400> 13

attgtagttc tggcattcc                                   19

<210> 14
<211> 20
<212> DNA
<213> artificial sequence

<220>
<223> probe N-P

<400> 14

cgttaggtcg gagcttgatc                                  20